# EUROPEAN PATENT APPLICATION

(11) **EP 3 777 853 A1**
(43) Date of publication of application: **17.02.2021**
(21) Application number: 19382711.0
(22) Date of filing: 14.08.2019
(51) Int. Cl.: A61K 31/426, A61K 9/20, A61K 47/38, A61P 3/10, A61P 13/00

(54) **MIRABEGRON COMPOSITION AND USES THEREOF**

(71) Applicant: BIOHORM, S.L., 08184 Palau Solita i Plegamans (Barcelona) (ES)
(72) Inventor: STRUSI, Orazio Luca, 08206 Sabadell (ES); CODERCH FERNÁNDEZ, Montserrat, 408172 Sant Cugat del Vallès (ES)
(74) Representative: Clarke Modet & Co.

(57) **Abstract**

Novel Mirabegron composition comprising: Mirabegron or a pharmaceutical salt thereof; HPMC; and MCC. The combination of HPMC and MCC provides for a controlled release of Mirabegron.

## Description

### Field of the invention

The present invention refers to the field of pharmaceuticals, more precisely to a pharmaceutical composition of Mirabegron capable of providing a controlled release thereof, more preferably, able to reduce food effects and with a release profile which is equivalent to that of the commercially available product Betmiga®.

### Background of the invention

(R)-2-(2-aminothiazol-4-yl)-4'-[2-[(2-hydroxy-2-phenylethyl)amino]ethyl]acetic acid anilide (hereinafter, Mirabegron) has been reported to have an activity of promoting insulin secretion, an activity of enhancing insulin sensitivity, an antiobesic activity and an antihyperlipemic activity based on an activity of selectively stimulating a β3 receptor.

On the basis of said activities it has been reported that the compound can be used as a therapeutic agent for the treatment of diabetes and overactive bladder, such as overactive bladder accompanied by prostatic hyperplasia, or overactive bladder accompanied by urinary urgency, urinary incontinence, and urinary frequency.

In Europe, Mirabegron has been approved by the European Medicines Agency for the symptomatic treatment of urgency, increased micturition frequency and/or urgency incontinence as may occur in adult patients with overactive bladder (OAB) syndrome.

It is known in the state of the art that Mirabegron, in the form of conventional formulations has disadvantages, for example, that pharmacokinetic data varies according to the presence or absence of the intake of food. For example, the rate of decrease of Cmax in a fed state was 67%, and the rate of decrease of AUC (Area Under the Curve) in the fed state was 47%, in comparison with those in a fasted state. These problems are considered to be raised by, for example, the changes in pharmacokinetics caused by food, and therefore, the development of a formulation capable of avoiding the effects by food intake has been a focus in the field.

Mirabegron is currently commercialized under the trade name of Betmiga®, being provided in the form of a composition that provides a controlled release of Mirabegron in a manner which allows to reduce the above-mentioned food effects. Betmiga® are tablets with 25 mg or 50 mg of Mirabegron and wherein the controlled-release of said active ingredient is achieved by means of a mixture of two polyethylene oxide of different molecular weight (also known as macrogol or polyethylene glycol) and hydroxypropyl cellulose and requiring butylhydroxytoluene as antioxidant.

The food effect problem with regard to Mirabegron also appears addressed in the PCT Patent application with publication number WO2017/186598A1, wherein a composition is disclosed comprising a therapeutically effective dose of mirabegron and a mixture of one or more polyethylene oxides, in order to achieve a controlled release.

Both, Betmiga® and WO2017/186598A1 use polyethylene oxides which, however, are prone to degradation due to oxidation, hence, providing for alterations in the controlled release of Mirabegron (leading to tablets with a non-acceptable behaviour) and variability between tablets and batches. WO2017/186598A1 already recognizes this problem and suggests adding an antioxidant to the composition and, as noted above, Betmiga® uses an antioxidant, increasing, hence, the complexity and variability of the composition.

Therefore, in view of the above problems, there is the need for compositions of Mirabegron which are simpler (and, hence, provide for decreased variability and increased robustness), avoid the use of polyethylene oxide (and, subsequently, of an antioxidant) and provide for a controlled release of Mirabegron. Preferably, the controlled release of Mirabegron is similar or identical to that provided by Betmiga®, leading, therefore, to a drug deemed to be bioequivalent to Betmiga®.

The inventors of the present invention, after extensive and exhaustive research, have surprisingly found that a composition comprising Mirabegron; hydroxypropyl methylcellulose (hereinafter, HPMC); and microcrystalline cellulose (hereinafter, MCC) solves the above-mentioned problems present in the state of the art. The composition found by the inventors of the present invention is a simple composition with low variability and increased robustness with regard to the compositions available in the state of the art, and still provide for a controlled release of Mirabegron. Moreover, the compositions according to the present invention do not require antioxidants as an additional excipient. In addition, the composition of the present invention surprisingly allows even to obtain tablets with a lower weight to that of the Mirabegron tablets available in the state of the art, providing hence, tablets which will be better accepted by the patients and which allow for manufacturing at lower costs due to a lower need of resources.

Moreover, in some preferred embodiments, the composition of the present invention has an in vitro release profile of Mirabegron which is similar or identical to that of Betmiga®. Such a similar in vitro release profile is normally a good indication that the resulting product will be bioequivalent to Betmiga®.

### Description of the invention

Therefore, in a first embodiment, the present invention refers to a composition of Mirabegron.

In addition, in a second embodiment, the present invention refers to a process for manufacturing a composition of the present invention.

In a third aspect, the present invention refers to the composition of the present invention for use as a medicament.

In a fourth aspect, the present invention refers to the composition of the present invention for use in the prevention, amelioration and/or treatment of overactive bladder or diabetes.

In a fifth aspect, the present invention refers to the use of the composition of the present invention for the manufacture of a medicament for the prevention, amelioration and/or treatment of overactive bladder or diabetes.

In a final aspect, the present invention refers to a method for the prevention, amelioration and/or treatment of overactive bladder or diabetes comprising administering the composition of the present invention to a subject in need thereof.

As used herein, (R)-2-(2-aminothiazol-4-yl)-4'-[2-[(2-hydroxy-2-phenylethyl)amino]ethyl]acetic acid anilide and Mirabegron are equivalent and are used herein interchangeably.

As used herein, "controlled release" means any controlled, sustained, prolonged, maintained or any other retarded release which is not considered being an immediate release.

As noted above, in a first aspect, the present invention refers to a Mirabegron composition, said composition comprising:
- Mirabegron or a pharmaceutical salt thereof;
- HPMC; and
- MCC.

In this composition, the excipients HPMC and MCC provide the controlled release of Mirabegron and the composition does not comprise any other component providing for said controlled release of Mirabegron.

In this first aspect, the present invention preferably refers to a Mirabegron composition, the composition consisting essentially of:
- Mirabegron or a pharmaceutical salt thereof;
- HPMC; and
- MCC.

Preferably, the composition comprises at least one type of HPMC with a specific viscosity. More preferably, the composition comprises only one type of HPMC with a specific viscosity. However, this invention also contemplates the possible use of two or more types of HPMC having each different viscosities in order to provide a mixture of HPMC with one specific global viscosity not available commercially.

The composition of the present invention is preferably a pharmaceutical composition.

In addition, preferably, the composition of the present invention is for the controlled release of Mirabegron. Therefore, more preferably, the composition of the present invention is a pharmaceutical composition for the controlled release of Mirabegron.

In the composition of the present invention the HPMC has a viscosity of between 10 mPa^{∗}s and 500 mPa^{∗}s, as measured in a 2% (weight/volume, hereinafter w/v; corresponds to 2 grams of HPMC in 100 mL of aqueous solution) aqueous solution of said HPMC at 20 °C. Preferably, the viscosity is from 50 mPa^{∗}s to 500 mPa^{∗}s, more preferably, from 50 mPa^{∗}s to 250 mPa^{∗}s, even more preferably from 75 mPa^{∗}s to 200 mPa^{∗}s, even more preferably from 75 mPa^{∗}s to 125 mPa^{∗}s, wherein the viscosity is as measured in a 2% (w/v) aqueous solution of said HPMC at 20 °C. Even more preferably, said HPMC has a viscosity of 100 mPa^{∗}s, as measured in a 2% (w/v) aqueous solution of said HPMC at 20 °C. In particular, the viscosity may be any value between 75 and 125 mPa^{∗}s, such as 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125 mPa^{∗}s and any combination thereof, as measured in a 2% (w/v) aqueous solution of said HPMC at 20 °C.

Also preferably, in the composition of the present invention the MCC is an MCC having a determined particle size. Preferably, the particle size, is provided as particle size distribution D50 (PSD D50), which is the median diameter of the particle size distribution. In the composition, the PSD D50 of MCC is less than 500 µm, preferably less than 400 µm, more preferably less than 350 µm, and even more preferably less than 250 µm. In another aspect, MCC has a PSD D50 of between 1 µm and 350 µm, preferably between 10 µm and 250 µm, more preferably of between 30 µm and 210 µm, even more preferably of between 50 µm and 180 µm, and even more preferably of between 70 µm and 110 µm. In one particular aspect, the PSD D50 is about 90 µm. In particular, the PSD D50 may be any value between 70 and 110 µm, such as 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110 µm, and any combination thereof.

The total weight of the composition is between 1.5 and 10 times the quantity of Mirabegron in the composition. Preferably, the total weight of the composition is between 1.5 and 9 times the quantity of Mirabegron in the composition. More preferably, the total weight of the composition is between 1.5 and 8.5 times the quantity of Mirabegron in the composition. Even more preferably, the total weight of the composition is between 1.5 and 8 times the quantity of Mirabegron in the composition. And even more preferably, the total weight of the composition is between 2 and 7.5 times the quantity of Mirabegron in the composition. In one embodiment, the total weight of the composition is between 3.5 and 4.8 times the quantity of Mirabegron in the composition. In particular, the total weight of the composition may be any weight that is 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0 times the quantity of Mirabegron in the composition, and any combination thereof.

Also in an embodiment, in the composition of the present invention the quantity of Mirabegron is between 10 and 200 mg. Preferably, the quantity of Mirabegron in the composition of the present invention is between 25 mg and 100 mg, more preferably, the quantity of Mirabegron in the composition of the present invention is between 25 mg and 50 mg, even more preferably, the quantity of Mirabegron in the composition of the present invention is 25 mg or 50 mg.

In a further embodiment of the present invention, the proportion by weight of MCC to HPMC may be MCC:HPMC from 1:9 to 1:0.11, preferably from 1:2.33 to 1:0.43, more preferably from 1:1.5 to 1:0.67, even more preferably from 1:1.5 to 1:0.82, and even more preferably from 1:1.5 to 1:1. In particular, the proportion by weight of MCC:HPMC may be any value between 1:0.82, 1:0.85, 1:0.89, 1:0.92, 1:0.96, 1:1, 1:1.02, 1:1.03, 1:1.04, 1:1.08, 1:1.13, 1:1.17, 1:1.22, 1:1.27, 1:1.30, 1:1.33, 1:1.38, 1:1.44, 1:1.5 and any combination thereof.

In an additional embodiment, in the composition of the present invention the proportion by weight of Mirabegron to HPMC may be Mirabegron:HPMC from 1:9 to 1:0.11, preferably from 1:5.67 to 1:0.67, even more preferably from 1:5.67 to 1:1, even more preferably from 1:5.67 to 1:1.22, and even more preferably from 1:4 to 1:1.22. In particular, the proportion by weight of Mirabegron:HPMC may be any value between 1:4, 1:3.76, 1:3.55, 1:3.35, 1:3.34, 1:3.17, 1:3, 1:2.99, 1:2.85, 1:2.70, 1:2.57, 1:2.45, 1:2.33, 1:2.23, 1:2.13, 1:2.03, 1:1.94, 1:1.86, 1:1.78, 1:1.70, 1:1.67, 1:1.63, 1:1.56, 1:1.5, 1:1.44, 1:1.38, 1:1.33, 1:1.28, 1:1.22 and any combination thereof .

It is contemplated that the composition of the present invention may comprise additional excipients, preferably pharmaceutically acceptable excipients. Said excipients are, preferably, selected from dyes, diluents, binders, lubricants, coating agents, glidants, flavours, disintegrants, sweeteners and combinations thereof.

It is also contemplated that the composition of the present invention is suitable for any route of administration. Therefore, the composition of the present invention can be in any form known in the state of the art and which is suitable for the elected route of administration. Preferably, the composition of the present invention is administered by parenteral route, intravenous route or by means of oral administration, more preferably, by means of oral administration.

Preferably, the composition of the present invention is manufactured into an oral dosage form, such as tablets, capsules, pellets, granules, powders, or films. Preferably, the composition of the present invention is provided in the form of a tablet.

In addition, the tablet can be of any shape know in the state of the art. Preferably, the tablet has an oblong or round shape, wherein the size of the round or oblong tablet depends on the total weight of composition.

Generally, when the tablet has a round shape it may have a, diameter from 5 to 15 mm. And when the tablet has an oblong shape, it may have a length from 5 to 25 mm and a width from 4 to 10 mm. However, depending on the total weight of the tablet, the dimension may be adapted accordingly and may thus be broader or narrower as mentioned above.

It is contemplated that the composition of the present invention comprises Mirabegron or a pharmaceutically acceptable salt thereof; HPMC; and MCC.

Preferably, the composition of the present invention comprises Mirabegron or a pharmaceutically acceptable salt thereof; HPMC; MCC; and one or more additional excipients selected from dyes, diluents, binders, lubricants, coating agents, glidants, flavours, disintegrants, sweeteners and combinations thereof. In this sense, in some embodiments, the composition of the present invention comprises Mirabegron or a pharmaceutically acceptable salt thereof; HPMC; MCC; and a lubricant. In other embodiments, the composition of the present invention comprises Mirabegron or a pharmaceutically acceptable salt thereof; HPMC; MCC; and a coating agent. More preferably, the composition of the present invention comprises Mirabegron or a pharmaceutically acceptable salt thereof; HPMC; MCC; a coating agent; and a lubricant.

Preferably, the compositions comprises at least one type of HPMC. More preferably, the composition comprises only one type of HPMC

It is also contemplated that in an embodiment the composition of the present invention may consist of Mirabegron or a pharmaceutically acceptable salt thereof; one type of HPMC; and MCC.

However, preferably, in further embodiments the composition of the present invention may consist of Mirabegron or a pharmaceutically acceptable salt thereof; one type of HPMC; MCC; and one or more additional excipients selected from dyes, diluents, binders, lubricants, coating agents, glidants, flavours, disintegrants, sweeteners and combinations thereof. In this sense, in some embodiments, the composition of the present invention may consist of Mirabegron or a pharmaceutically acceptable salt thereof; one type of HPMC; MCC; and a lubricant. In other embodiments, the composition of the present invention may consist of Mirabegron or a pharmaceutically acceptable salt thereof; one type of HPMC; MCC; and a coating agent. In still other embodiments, the composition of the present invention may consist of Mirabegron or a pharmaceutically acceptable salt thereof; one type of HPMC; MCC; a coating agent; and a lubricant.

Moreover, it is preferred that the composition of the present invention does not comprise any antioxidant.

The combination of HPMC (preferably, one type of HPMC) and the MCC provide for the controlled release of Mirabegron. In addition, the fact that only said two components are required for the controlled release of Mirabegron provides for:
- A simpler composition.
- Avoids the use of polyethylene oxides and, hence the need for antioxidants.
- It is easier to control the controlled release of Mirabegron as it only depends on the combination of HPMC and MCC.
- Low variability.
- Increased robustness.

Therefore, the composition of the present invention solves the technical problems present in the state of the art and mentioned above.

As noted above and below in the examples, in the preferred embodiment of the present invention, the composition further provides for a Mirabegron in vitro release profile which is similar or identical to that of Betmiga®. In addition, the inventors also have surprisingly found that the simple composition of the present invention has the additional technical effect that tablets with a lower weight (and, hence, smaller) to that of Betmiga® can be obtained. Tablets with lower weight are advantageous because less resources are needed in order to manufacture such tablets, whereas smaller dimensions increase patience's acceptance, since smaller tablets are easier to swallow. This additional technical effects are achieved when:
- The total weight of the composition is less than 250 mg (this is, less than the weight of Betmiga®, which is about 250 mg without coating and about 257 mg with coating), preferably the total weight of the composition is equal to or less than 240 mg, more preferably, equal to or less than 220 mg, and even more preferably equal to or less than 200 mg.
- The total weight of the composition is between 1.5 and 9 times the quantity of Mirabegron, more preferably, between 2 and 8 times the quantity of Mirabegron. More preferred ranges depend on the total amount of Mirabegron present in the composition and may, without limitations, for example be any range from 2 to 7, 3 to 7, 3.5 to 7.5, 3.5 to 7, or 3.5 to 5 times the quantity of Mirabegron, and any combinations thereof. In particular, the total weight of the composition may be any weight that is 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0 times the quantity of Mirabegron, and any combinations thereof.

In this preferred embodiment, preferably the proportion by weight of Mirabegron to HPMC is Mirabegron:HPMC from approximately 1:3.34 to approximately 1:1, more preferably from 1:3.34 to 1:1, even more preferably from approximately 1:3.34 to approximately 1:1.5.

Also in this preferred embodiment, the proportion by weight of MCC to HPMC is MCC:HPMC from approximately 1:1.30 to approximately 1:1.02, more preferably from 1:1.30 to 1:1.02.

In this preferred embodiment, preferably, the quantity of Mirabegron is 25 mg. In this case, the total weight of the composition is preferably between 1.5 and 9 times the quantity of Mirabegron in the composition and, hence, the total weight of the composition is between 37.5 mg and 225 mg. More preferably, the total weight of the 25 mg Mirabegron composition is between 2 and 8.5 times the quantity of Mirabegron in the composition and, hence, the total weight of the composition is between 50 mg and 212.5 mg. Even more preferably, the total weight of the 25 mg Mirabegron composition is between 3.5 and 8.5 times the quantity of Mirabegron in the composition and, hence, the total weight of the composition is between 87.5 mg and 212.5 mg. And even more preferably, the total weight of the 25 mg Mirabegron composition is between 3.5 and 8 times the quantity of Mirabegron in the composition and, hence, the total weight of the composition is between 87.5 mg and 200 mg. In even one aspect, the total weight of the 25 mg Mirabegron composition may be between 3.5 and 4.5 times the quantity of Mirabegron in the composition and, hence, the total weight of the composition is between 87.5 mg and 112.5 mg. Hence, tablets are obtained that have a low weight and small size, resulting in better patient acceptance and more economic manufacturing.

In one particular embodiment of the preferred embodiment mentioned above, when the quantity of Mirabegron is 25 mg the proportion by weight of MCC:HPMC is 1:1.02 and the proportion by weight of Mirabegron:HPMC is 1:2.99.

In a further particular embodiment of the preferred embodiment mentioned above, when the quantity of Mirabegron is 25 mg the proportion by weight of MCC:HPMC is 1:1.3 and the proportion by weight of Mirabegron:HPMC is 1:3.34.

In an additional particular embodiment of the preferred embodiment mentioned above, when the quantity of Mirabegron is 25 mg the proportion by weight of MCC:HPMC is 1:1.02 and the proportion by weight of Mirabegron:HPMC is 1:1.50.

In a further particular embodiment of the preferred embodiment mentioned above, when the quantity of Mirabegron is 25 mg the proportion by weight of MCC:HPMC is 1:1.3 and the proportion by weight of Mirabegron:HPMC is 1:1.67.

In the above-mentioned preferred embodiment, also preferably, the quantity of Mirabegron is 50 mg. In this case, the total weight of the composition is between 1.5 and 4.8 times the quantity of Mirabegron in the composition, more preferably the total weight of the composition is between 1.5 and 4.5 times the quantity of Mirabegron in the composition, even more preferably between 2 and 4.5 times the quantity of Mirabegron, and even more preferably between 2.5 and 4.2 times the quantity of Mirabegron. Therefore, the total weight of the composition is between 75 mg and 240 mg, preferably between 75 mg and 225 mg, even more preferably between 100 mg and 225 mg, and even more pre between 125 mg and 210 mg. In one aspect, the total weight of the composition may be between 3.5 and 4.5 times the quantity of Mirabegron in the composition, more preferably the total weight of the composition may be between 4 and 4.2 times the quantity of Mirabegron in the composition. In this case, the total weight of the composition may be between 175 mg and 225 mg, preferably between 200 mg and 210 mg.

In this case, preferably the proportion by weight of Mirabegron:HPMC is from approximately 1:1 to approximately 1:1.67 (preferably, from approximately 1:1.5 to approximately 1:1.67) and the proportion by weight of MCC:HPMC is from approximately 1:1.02 to approximately 1:1.28.

In a most preferred embodiment of the preferred embodiment mentioned above, when the quantity of Mirabegron is 50 mg the proportion by weight of MCC:HPMC is 1:1.02 and the proportion by weight of Mirabegron:HPMC is 1:1.50.

In another particular embodiment of the preferred embodiment mentioned above, when the quantity of Mirabegron is 50 mg the proportion by weight of MCC:HPMC is 1:1.03 and the proportion by weight of Mirabegron:HPMC is 1:1.

In one particular embodiment of the preferred embodiment mentioned above, when the quantity of Mirabegron is 50 mg the proportion by weight of MCC:HPMC is 1:1.28 and the proportion by weight of Mirabegron:HPMC is 1:1.67.

In the case of the preferred embodiment and the most preferred embodiments explained thereof, all the embodiments and features explained above for the composition of the present invention apply or are applicable as long as they do not contradict a feature specified in said preferred embodiment and/or in the most preferred embodiments explained thereof.

Regarding the preferred embodiment and the most preferred embodiments explained thereof, it is noted that they also provide for the technical effects mentioned above for the composition of the present invention. In addition, they also provide for the following surprising and unexpected technical effects:
- An in vitro release profile of Mirabegron which is similar or identical to that of Betmiga®.
- Tablets which have lower weight and are smaller to those commercially available (Betmiga®) and, even smaller to those disclosed in the state of the art, therefore, providing tablets which are produced more economically, which allow to use less material in their manufacture and which are better accepted by the patients.

In a second embodiment, the present invention refers to a process for manufacturing a composition of the present invention.

In a third aspect, the present invention refers to the composition of the present invention for use as a medicament.

The composition of the present invention is as explained above.

The composition of the present invention is used in a subject in need thereof, preferably a mammal, even more preferably a human.

The composition of the present invention is used at a pharmaceutically effective dose. Preferably, the composition of the present invention provides for a pharmaceutically effective dose of Mirabegron, more preferably, a dose of 25 mg or 50 mg.

The composition of the present invention can be administered by any means or route known in the state of the art and it will be suited for said administration route. Preferably, the administration route is parenteral, intravenous or oral, more preferably, the administration route is oral.

The oral dosage form obtained from the composition of the invention can be provided in any packaging and depending on the regulatory requirements of the country or region where the tablets shall be distributed. For example, the oral dosage form may be provided in blister material or bottles. Blister material includes all the known materials, such as for example and without limitation Alu/Alu (aluminium/aluminium), PVC/Alu (Polyvinylchloride/aluminium), PVC/PCTFE/Alu (Polyvinylchloride/ Polychlorotrifluoroethylene/aluminium), Polyamide/Alu/PVC-Alu (Polyamide/Aluminium/Polyvinyl chloride-aluminium) or PVC/PVDC/Alu (Polyvinyl chloride/ Polyvinylidene chloride/aluminium), Bottles may be made, without limitations, from glass, or plastic material, for example HDPE (High-density polyethylene), LDPE (Low-density polyethylene), or PET (Polyethylene terephthalate).

In a fourth aspect, the present invention refers to the composition of the present invention for use in the prevention, amelioration and/or treatment of overactive bladder or diabetes.

The composition of the present invention is as explained above.

The composition of the present invention is used in a subject in need thereof, preferably a mammal, even more preferably a human.

The composition of the present invention is used at a pharmaceutically effective dose. Preferably, the composition of the present invention provides for a pharmaceutically effective dose of Mirabegron, more preferably, a dose of 25 mg or 50 mg.

The composition of the present invention can be administered by any means or route known in the state of the art and it will be suited for said administration route. Preferably, the administration route is parenteral intravenous route or oral, more preferably, the administration route is oral.

The oral dosage form obtained from the composition of the invention can be provided in any packaging and depending on the regulatory requirements of the country or region where the tablets shall be distributed. For example, the oral dosage form may be provided in blister material or bottles. Blister material includes all the known materials, such as for example and without limitation Alu/Alu (aluminium/aluminium), PVC/Alu (Polyvinylchloride/aluminium), PVC/PCTFE/Alu (Polyvinylchloride/ Polychlorotrifluoroethylene/aluminium), Polyamide/Alu/PVC-Alu (Polyamide/Aluminium/Polyvinyl chloride-aluminium) or PVC/PVDC/Alu (Polyvinyl chloride/ Polyvinylidene chloride/aluminium), Bottles may be made, without limitations, from glass, or plastic material, for example HDPE (High-density polyethylene), LDPE (Low-density polyethylene), or PET (Polyethylene terephthalate).

Preferably, the composition of the present invention is for use in the treatment of overactive bladder or diabetes.

Also preferably, the overactive bladder is overactive bladder accompanied by prostatic hyperplasia, or overactive bladder accompanied by urinary urgency, urinary incontinence, and urinary frequency.

In a fifth aspect, the present invention refers to the use of the composition of the present invention for the manufacture of a medicament for the prevention, amelioration and/or treatment of overactive bladder or diabetes.

The composition of the present invention is as explained above.

The composition of the present invention is used in a subject in need thereof, preferably a mammal, even more preferably a human.

The composition of the present invention is used at a pharmaceutically effective dose. Preferably, the composition of the present invention provides for a pharmaceutically effective dose of Mirabegron, more preferably, a dose of 25 mg or 50 mg.

The composition of the present invention can be administered by any means or route known in the state of the art and it will be suited for said administration route. Preferably, the administration route is parenteral intravenous route or oral, more preferably, the administration route is oral.

The oral dosage form obtained from the composition of the invention can be provided in any packaging and depending on the regulatory requirements of the country or region where the tablets shall be distributed. For example, the oral dosage form may be provided in blister material or bottles. Blister material includes all the known materials, such as for example and without limitation Alu/Alu (aluminium/aluminium), PVC/Alu (Polyvinylchloride/aluminium), PVC/PCTFE/Alu (Polyvinylchloride/ Polychlorotrifluoroethylene/aluminium), Polyamide/Alu/PVC-Alu (Polyamide/Aluminium/Polyvinyl chloride-aluminium) or PVC/PVDC/Alu (Polyvinyl chloride/ Polyvinylidene chloride/aluminium), Bottles may be made, without limitations, from glass, or plastic material, for example HDPE (High-density polyethylene), LDPE (Low-density polyethylene), or PET (Polyethylene terephthalate).

Preferably, the use of the composition of the present invention for the manufacture of a medicament is for the treatment of overactive bladder or diabetes.

Also preferably, the overactive bladder is overactive bladder accompanied by prostatic hyperplasia, or overactive bladder accompanied by urinary urgency, urinary incontinence, and urinary frequency.

In another aspect, the present invention refers to a method for the prevention, amelioration and/or treatment of overactive bladder or diabetes comprising administering the composition of the present invention to a subject in need thereof.

The composition of the present invention is as explained above.

The subject in need thereof is, preferably, a mammal, even more preferably a human.

The composition of the present invention is used at a pharmaceutically effective dose. Preferably, the composition of the present invention provides for a pharmaceutically effective dose of Mirabegron, more preferably, a dose of 25 mg or 50 mg.

The composition of the present invention is administered by any means or route known in the state of the art and it will be suited for said administration route. Preferably, the administration route is parenteral intravenous route or oral, more preferably, the administration route is oral.

The oral dosage form obtained from the composition of the invention can be provided in any packaging and depending on the regulatory requirements of the country or region where the tablets shall be distributed. For example, the oral dosage form may be provided in blister material or bottles. Blister material includes all the known materials, such as for example and without limitation Alu/Alu (aluminium/aluminium), PVC/Alu (Polyvinylchloride/aluminium), PVC/PCTFE/Alu (Polyvinylchloride/ Polychlorotrifluoroethylene/aluminium), Polyamide/Alu/PVC-Alu (Polyamide/Aluminium/Polyvinyl chloride-aluminium) or PVC/PVDC/Alu (Polyvinyl chloride/ Polyvinylidene chloride/aluminium), Bottles may be made, without limitations, from glass, or plastic material, for example HDPE (High-density polyethylene), LDPE (Low-density polyethylene), or PET (Polyethylene terephthalate).

Preferably, the method is for the treatment of overactive bladder or diabetes.

Also preferably, the overactive bladder is overactive bladder accompanied by prostatic hyperplasia, or overactive bladder accompanied by urinary urgency, urinary incontinence, and urinary frequency.

Hereinafter, the present invention will be explained or disclosed with reference to the following statements
Statement 1: Composition comprising:
   - Mirabegron or a pharmaceutical salt thereof;
   - HPMC; and
   - MCC.
Statement 2: Composition comprising:
   - Mirabegron or a pharmaceutically acceptable salt thereof;
   - one type of HPMC; and
   - MCC.
Statement 3: Composition in accordance with statement 1 or statement 2, characterized in that the HPMC and the MCC are in charge of providing the controlled release of Mirabegron and wherein the composition does not comprise any other component in charge of providing the controlled release of Mirabegron.
Statement 4: Composition consisting essentially of:
   - Mirabegron or a pharmaceutically acceptable salt thereof;
   - HPMC; and
   - MCC.
Statement 5: Composition consisting essentially of:
   - Mirabegron or a pharmaceutically acceptable salt thereof;
   - one type of HPMC; and
   - MCC.
Statement 6: Composition in accordance with any one of statements 1 to 5, characterized in that the HPMC has a viscosity of between 10 mPa^{∗}s and 500 mPa^{∗}s in a 2% (w/v) aqueous solution of said HPMC at 20 °C.
Statement 7: Composition in accordance with any one of statements 1 to 6, characterized in that HPMC has a viscosity of 100 mPa^{∗}s in a 2% (w/v) aqueous solution of said HPMC at 20 °C.
Statement 8: Composition in accordance with any one of the statements 1 to 7, characterized in that the MCC has a PSD D50 of less than 500 µm.
Statement 9: Composition in accordance with any one of the statements 1 to 8, characterized in that the MCC has a PSD D50 of between 1 and 350 µm.
Statement 10: Composition in accordance with any one of the statements 1 to 9, characterized in that the total weight of the composition is between 1.5 and 10 times the quantity of Mirabegron in the composition.
Statement 11: Composition in accordance with statement 10, characterized in that the total weight of the composition is between 1.5 and 9 times the quantity of Mirabegron in the composition.
Statement 12: Composition in accordance with any one of the statements 1 to 11, characterized in that the quantity of Mirabegron is between 10 and 200 mg, preferably the quantity of Mirabegron is 25 mg or 50 mg.
Statement 13: Composition in accordance with any one of the statements 1 to 12, characterized in that the proportion by weight of MCC to HPMC is MCC:HPMC from approximately 1:9 to approximately 1:0.11.
Statement 14: Composition in accordance with any one of the statements 1 to 13, characterized in that the proportion by weight of Mirabegron to HPMC is Mirabegron:HPMC from approximately 1:9 to approximately 1:0.11.
Statement 15: Composition in accordance with any one of the statements 1 to 14, characterized in that:
   - the total weight of the composition is less than 250 mg, and
   - the total weight of the composition is between 1.5 and 9 times the quantity of Mirabegron.
Statement 16: Composition in accordance with statement 15, characterized in that the total weight of the composition is between 2 and 8 times the quantity of Mirabegron. Statement 17: Composition in accordance with any of statement 15 or statement 16, characterized in that the proportion by weight of Mirabegron to HPMC is Mirabegron:HPMC from approximately 1:3.34 to approximately 1:1.
Statement 18: Composition in accordance with any of the statements 15 to 17, characterized in that the proportion by weight of MCC to HPMC is MCC:HPMC from approximately 1:1.30 to approximately 1:1.02.
Statement 19: Composition in accordance with any one of statements 1 to 18, characterized in that the quantity of Mirabegron is 25 mg.
Statement 20: Composition in accordance with statement 19, characterized in that the total weight of the composition is between 37.5 mg and 225 mg.
Statement 21: Composition in accordance with any one of statements 1 to 18, characterized in that the quantity of Mirabegron is 50 mg.
Statement 22: Composition in accordance with statement 21, characterized in that the total weight of the composition is between 1.5 and 4.8 times the quantity of Mirabegron in the composition and, hence, the total weight of the composition is between 75 mg and 240 mg.
Statement 23: Composition in accordance with any one of the statements 21 to 22, characterized in that the proportion by weight of Mirabegron to HPMC is Mirabegron:HPMC from approximately 1:1.67 to approximately 1:1.
Statement 24: Composition in accordance with any one of the statements 21 to 23, characterized in that the proportion by weight of MCC to HPMC is MCC:HPMC from approximately 1:1.28 to approximately 1:1.02.
Statement 25: Composition in accordance with any one of the statements 1 to 24, characterized in that it further comprises additional excipients.
Statement 26: Composition in accordance with statement 25, characterized in that the additional excipients are selected from the group consisting of dyes, diluents lubricants, coating agents, glidants, flavours, disintegrants, sweeteners and combinations thereof.
Statement 27: Composition in accordance with any one of statements 1 to 26, characterized in that the composition is a pharmaceutical composition.
Statement 28: Composition in accordance with any of statements 1 to 27, characterized in that the composition is in the form of a tablet, preferably having an oblong or round shape.
Statement 29: Composition in accordance with any one of statements 1 to 28, characterized in that it consists of Mirabegron or a pharmaceutically acceptable salt thereof; one type of HPMC; and MCC.
Statement 30: Composition in accordance with any one of statements 1 to 28, characterized in that it consists of Mirabegron or a pharmaceutically acceptable salt thereof; one type of HPMC; MCC; and a lubricant.
Statement 31: Composition in accordance with any one of statements 1 to 28, characterized in that it consists of Mirabegron or a pharmaceutically acceptable salt thereof; one type of HPMC; MCC; and a coating agent.
Statement 32: Composition in accordance with any one of statements 1 to 28, characterized in that it consists of Mirabegron or a pharmaceutically acceptable salt thereof; one type of HPMC; MCC; a coating agent; and a lubricant.
Statement 33: Composition in accordance with any one of statements 1 to 32, characterized in that the composition is for the controlled release of Mirabegron. Statement 34: Process for the production of a composition in accordance with any one of statements 1 to 33.
Statement 35: Composition in accordance with any one of statements 1 to 33 for use as a medicament.
Statement 36: Composition in accordance with any one of statements 1 to 33 for use in the treatment of overactive bladder or diabetes
Statement 37: Use of the composition in accordance with any one of statements 1 to 33 for the manufacture of a medicament for the prevention, amelioration and/or treatment of overactive bladder or diabetes.
Statement 38: Method for the prevention, amelioration and/or treatment of overactive bladder or diabetes comprising administering a composition in accordance with any one of statements 1 to 33 to a subject in need thereof.

To allow a better understanding, the present invention is described in more detail below with reference to the enclosed drawings, which are presented by way of example, and with reference to illustrative and non-limitative examples.

In the following figures, release profiles are shown illustrating the percentage of Mirabegron dissolved from the tablet over time, using a dissolution test at pH 6.8. The x-axis in these figures refers to the time in minutes; and the y-axis refers to the percentage of Mirabegron dissolved (in weight and with regard to the total Mirabegron present in the tablet at the beginning of the assay).
Figure 1 shows the release profile of Mirabegron obtained for Betmiga®.
Figure 2 shows the release profile of Mirabegron obtained for composition 1 (see table 1 for details of the composition).
Figure 3 shows the release profile of Mirabegron obtained for composition 2 (see table 1 for details of the composition).
Figure 4 shows the release profile of Mirabegron obtained for composition 3 (see table 1 for details of the composition).
Figure 5 shows the release profile of Mirabegron obtained for composition 4 (see table 1 for details of the composition).
Figure 6 shows the release profile of Mirabegron obtained for composition 5 (see table 1 for details of the composition).
Figure 7 shows the release profile of Mirabegron obtained for composition 6 (see table 1 for details of the composition).
Figure 8 shows the release profile of Mirabegron obtained for composition 7 (see table 1 for details of the composition).
Figure 9 shows the release profile of Mirabegron obtained for composition 8 (see table 1 for details of the composition).
Figure 10 shows the release profile of Mirabegron obtained for composition 9 (see table 1 for details of the composition).
Figure 11 shows the release profile of Mirabegron obtained for composition 10 (see table 1 for details of the composition).
Figure 12 shows the release profile of Mirabegron obtained for composition 11 (see table 1 for details of the composition).

### Examples.

### Example 1. Preparation of tablets of the composition of the present invention.

Tablets according to the composition 1 (see table 1 below) of the present invention were prepared by means of manufacture procedures known in the state of the art.

In a first step, 663.65 g of HPMC 100 mPa^{∗}s and 223 g of Mirabegron were mixed and homogenized for 15 minutes at 25 revolutions per minute (hereinafter, rpm) to prepare a granulate of HPMC + Mirabegron.

An amount of 4.55 of HPMC 100 mPa^{∗}s (as measured in a 2% (w/v) aqueous solution of said HPMC at 20 °C) was dissolved in 227.45 g of purified water in order to prepare an HPMC solution. This solution was then used as a binder-to ensure that the granulate had an appropriate rheology and that no residues remained in the instruments used. The HPMC solution was prepared with an excess of about 13% by weight of HPMC with regard to the amount of HPMC that later was applied to the granulate. This was done in order to compensate some minor loss of the HPCM solution due to the equipment used during the spraying process.

The so prepared HPMC solution was sprayed over the HPMC + Mirabegron granulate inside a high shear granulator with 5,8 m/s of tip speed for 4.5 minutes with a 1.2 diameter nozzle.

Next, the obtained granulate was dried until it reached a relative humidity of approximately 8%.

Then, the granulate was sieved to a size of 3.175 mm and dried once again to reach a relative humidity of less than 4%.

Next, the obtained product was sieved once again to a size of 0.991 mm.

At this point, the granulate was mixed with the 653.39 g of MCC and 15.61 g of magnesium stearate were also added to ensure that the resulting composition did not adhere to any surface.

Then, the obtained composition was adequately shaped into tablets.

To coat the obtained tablets, Opadry 03F230046 orange was added to purified water and stirred during 45 min. The solution was then applied in a pan coater to the tablets (5.25 mg of Opadry 03F230046 orange per tablet).

### Example 2. Analysis of the controlled release of Mirabegron of tablets of the composition of the present invention.

The compositions 2 to 11 included in table 1 were prepared in accordance with the manufacturing procedure described in example 1 referring to composition 1.

**Table 1. Details of the composition of compositions 1 to 11.**

| **Composition** | **Mirabegron (mg)** | **HPMC (mg)** | **MCC (mg)** | **Others** | **Total (mg)** |
|---|---|---|---|---|---|
| Composition 1 | 25 | 74.85 | 73.25 | - Magnesium stearate: 1.75 mg. | 180.1 |
| | | | | - Opadry 03F230046 orange: 5.25 mg. | |
| Composition 2 | 50 | 74.85 | 73.25 | - Magnesium stearate: 1.75 mg. | 205.85 |
| | | | | - Opadry 03F220127 yellow: 6 mg. | |
| Composition 3 | 25 | 83.59 | 64.51 | - Magnesium stearate: 1.75 mg. | 180.1 |
| | | | | - Opadry 03F230046 orange: 5.25 mg. | |
| Composition 4 | 25 | 75 | 147.5 | - Magnesium stearate: 2.5 mg. | 250 |
| Composition 5 | 50 | 50 | 48.5 | - Magnesium stearate: 1.5 mg | 150 |
| Composition 6 | 50 | 150 | 146.50 | - Magnesium stearate: 3.5 mg. | 350 |
| Composition 7 | 50 | 149.7 | 146.5 | - Magnesium stearate: 3.5 mg. | 349.7 |
| Composition 8 | 50 | 167.48 | 129.02 | - Magnesium stearate: 3.5 mg. | 350 |
| Composition 9 | 25 | 37.425 | 36.625 | - Magnesium stearate: 0.875 mg. | 99.925 |
| Composition 10 | 25 | 41.795 | 32.255 | - Magnesium stearate: 0.875 mg. | 99.925 |
| Composition 11 | 50 | 83.74 | 65.51 | - Magnesium stearate: 1.75 mg | 201 |

Betmiga® used in this example was a 25 mg Mirabegron tablet commercially available.

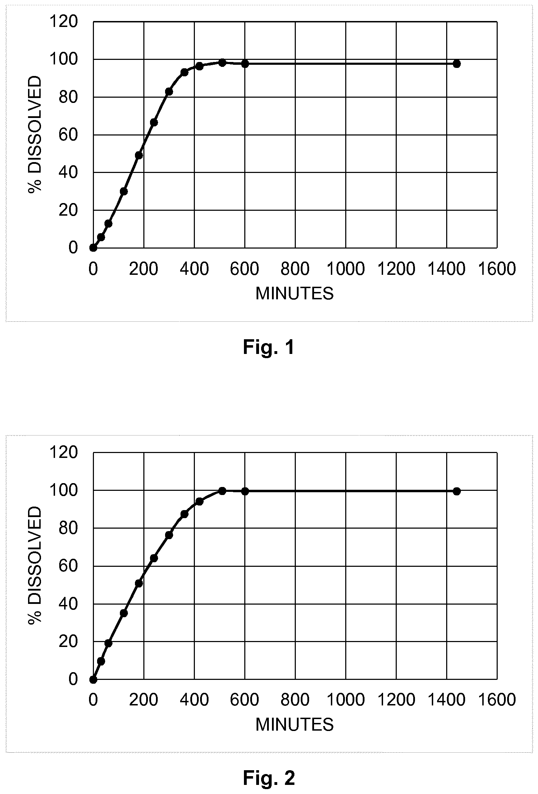

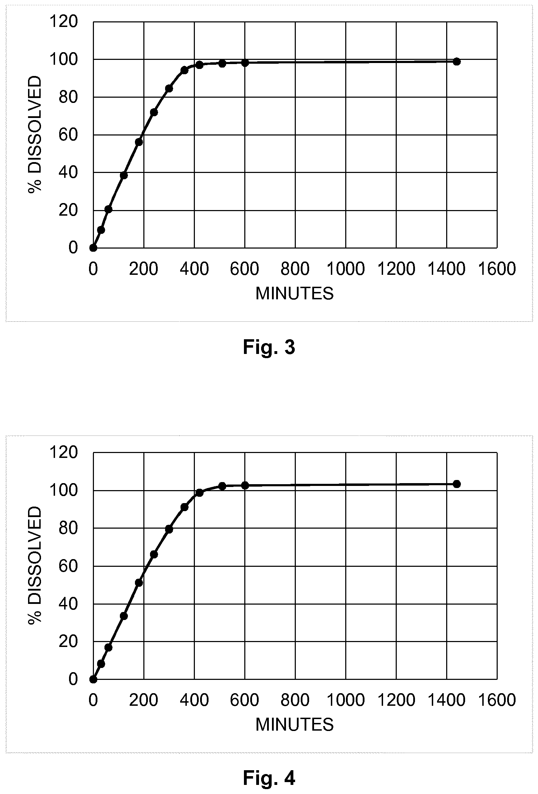

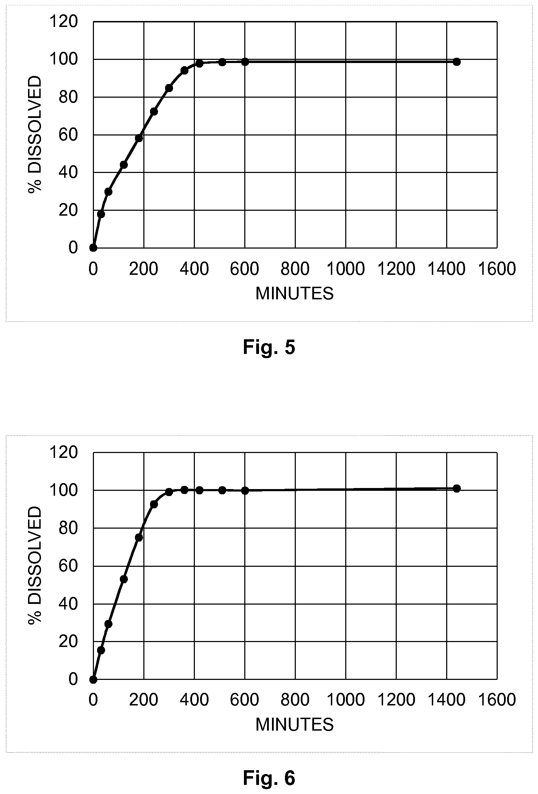

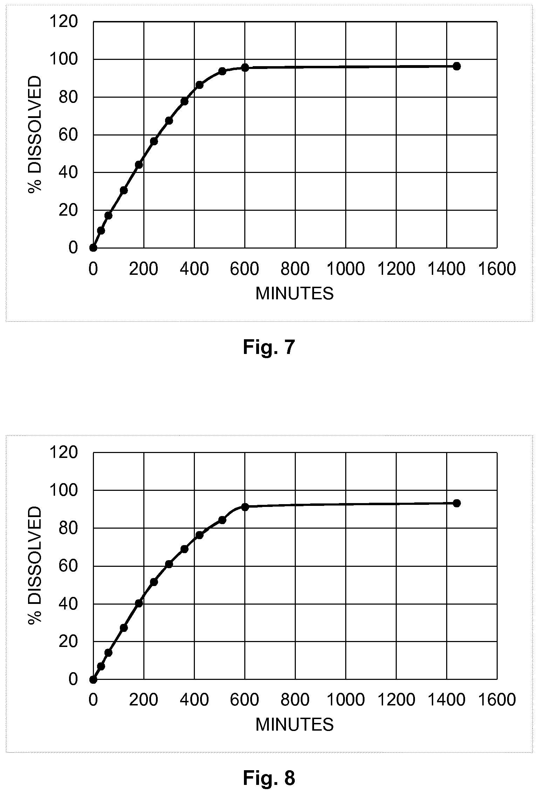

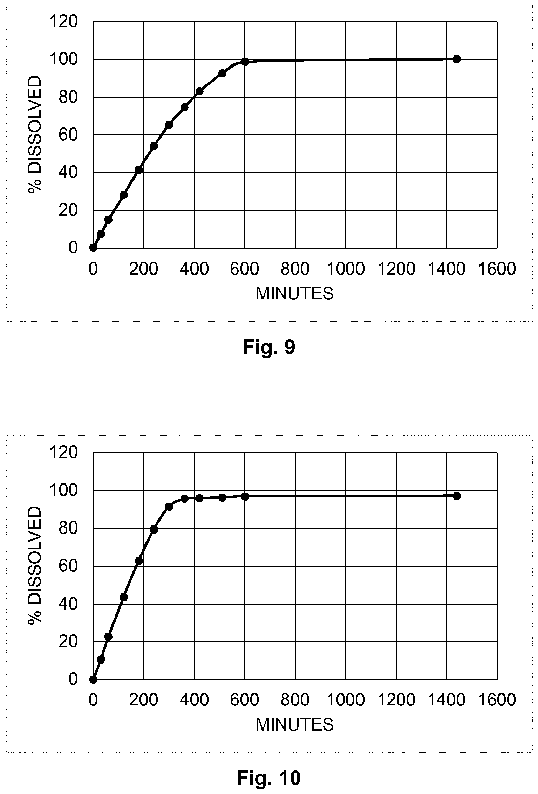

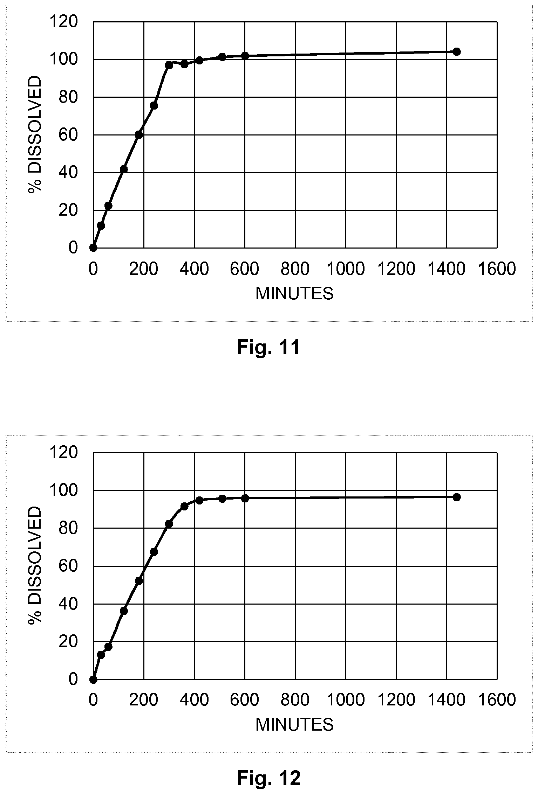

All the compositions noted in table 1 were tested for their ability to delay release of Mirabegron (controlled release of Mirabegron). To that end, the following dissolution test was carried out for each of the compositions of table 1 and a commercially available tablet of 25 mg of Mirabegron per tablet (Betmiga®) as mentioned above, and the amount of released Mirabegron was determined by UPLC (Ultrahigh Pressure Liquid Chromatography) using standard analysis techniques and the substance Mirabegron as reference:
- Dissolution test in 900 mL of a phosphate buffering solution at a pH of 6.8 at a temperature of about 37 °C ± 0.5 °C in Quadrangular Baskets with a stirring of 100 rpm.
The results obtained appear summarized figures 1 to 12.

As can be derived from Figures 2 to 12, compositions 1 to 11 showed an effective ability to delay release of Mirabegron (controlled release of Mirabegron).

Therefore, the composition of the present invention solves the technical problems present in the state of the art mentioned above, and, furthermore, provides for a simpler composition to allow the controlled release of Mirabegron, without using polyethylene oxides and necessary antioxidants and, hence, with low variability and increased robustness.

In addition, and surprisingly, when comparing Figures 2 to 12 and to the release results obtained for the table commercially available of 25 mg of Mirabegron (Betmiga®) (see figure 1), we can see that compositions 1 to 3 and 9 to 11 of the invention provide for tablets with a similar or identical Mirabegron in vitro release profile as Betmiga®. Therefore, the results obtained for compositions 1 to 3 and 9 to 11 are even more surprising as said compositions not only provide for an effective delay in the release of Mirabegron (controlled release of Mirabegron) but they also provided for a release profile which resembles that of Betmiga® allowing the manufacture and provision of bioequivalent compositions or drugs to Betmiga®.

Moreover, it is noted that the present invention allows to obtain tablets with reduced weight and reduced size with regard to Betmiga®, providing, hence, tablets which will be better accepted and tolerated by the patients and that allow for a more cost-efficient manufacture of Mirabegron-containing oral dosage forms, such as tablets.

## Claims

1. Composition comprising:
- Mirabegron or a pharmaceutical salt thereof;
- Hydroxypropyl methylcellulose (HPMC); and
- Microcrystalline cellulose (MCC),

2. Composition according to claim 1, **characterized in that** only one type of HPMC is used.

3. Composition in accordance with any one of claims 1 to 2, **characterized in that** the HPMC has a viscosity of between 10 mPa^{∗}s and 500 mPa^{∗}s in a 2% (w/v) aqueous solution of said HPMC at 20 °C.

4. Composition in accordance with any one of claims 1 to 3, **characterized in that** the MCC has a PSD D50 of less than500 µm.

5. Composition in accordance with any one of claims 1 to 4, **characterized in that** the quantity of Mirabegron is between 10 and 200 mg, preferably the quantity of Mirabegron is 25 mg or 50 mg.

6. Composition in accordance with any one of claims 1 to 5, **characterized in that** the proportion by weight of MCC to HPMC is Mirabegron:HPMC from approximately 1:9 to approximately 1:0.11.

7. Composition in accordance with any one of claims 1 to 6, **characterized in that** the proportion by weight of Mirabegron to HPMC is Mirabegron:HPMC from approximately 1:9 to approximately 1:0.11.

8. Composition in accordance with any one of claims 1 to 7, **characterized in that** it further comprises additional excipients.

9. Composition in accordance with claim 8, **characterized in that** the additional excipients are selected from the group consisting of dyes, diluents, binders, lubricants, coating agents, glidants, flavours, disintegrants, sweeteners and combinations thereof.

10. Composition in accordance with any of claims 1 to 9, **characterized in that** the composition is in the form of an oral dosage form, preferably in the form of a tablet.

11. Composition in accordance with any one of claims 1 to 10, **characterized in that**:
- the total weight of the composition is less than 250 mg, and
- the total weight of the composition is between 1.5 and 9 times the quantity of Mirabegron.

12. Process for manufacturing a composition in accordance with any one of claims 1 to 11.

13. Composition in accordance with any one of claims 1 to 11 for use as a medicament.

14. Composition in accordance with any one of claims 1 to 11 for use in the prevention, amelioration and/or treatment of overactive bladder or diabetes.
